# EUROPÄISCHE PATENTSCHRIFT

(11) **EP 1 474 142 B1**
(45) Veröffentlichungstag und Bekanntmachung des Hinweises auf die Patenterteilung: **28.04.2010**
(21) Anmeldenummer: 03709707.8
(22) Anmeldetag: 14.02.2003
(51) Int. Cl.: A61K 31/4415, A61K 31/714, A61K 31/52, A61P 3/02, A61P 7/02, A61K 9/48, A61K 9/20

(54) **MITTEL ENTHALTEND FOLSÄURE, VITAMIN B6 UND VITAMIN B12, UND DESSEN VERWENDUNG**
PREPARATIONS CONTAINING FOLIC ACID, VITAMIN B6, AND VITAMIN B12, AND USE THEREOF
PRODUITS CONTENANT DE L'ACIDE FOLIQUE, DE LA VITAMINE B6 ET DE LA VITAMINE B12, ET SON UTILISATION

(30) Priorität: 14.02.2002 DE 10206159
(43) Veröffentlichungstag der Anmeldung: 10.11.2004
(73) Patentinhaber: Synervit Ltd., Grants Hill Ilford, Essex IG2 6HY (GB)
(72) Erfinder: GÖRNE, Martin, 22337 Hamburg (DE)
(74) Vertreter: Reitstötter - Kinzebach
(86) Internationale Anmeldenummer: PCT/EP2003/001505
(87) Internationale Veröffentlichungsnummer: WO 2003/068231

(56) Entgegenhaltungen:
- US-A- 6 129 918
- US-B1- 6 297 224

## Beschreibung

Die vorliegende Erfindung betrifft Mittel, enthaltend Folsäure, Vitamin B6 und Vitamin B12, und deren Verwendung zur Regulation von Homocysteinspiegeln. Die Mittel sind damit vor allem zur vorbeugenden und akuten Behandlung von vaskulären Erkrankungen brauchbar. Beschrieben werden insbesondere pharmazeutische Mittel und Nahrungsergänzungsmittel mit einer entsprechenden Wirkstoffkombination sowie Mittel in Form von Handelspackungen mit entsprechenden Kombinationspräparaten oder Monopräparaten zur kombinierten Anwendung.

Seit kurzem ist bekannt, dass Homocystein ein Risikofaktor für konorare, periphere und zerebrale vaskuläre Erkrankungen darstellt. Patienten mit heriditärer Hyperhomocysteinämie, einer autosomal rezessiven Erkrankung, haben Homocystein-Plasmaspiegel, die etwa 10-20-fach über den Normalwerten liegen. Bei Kindern, die von der homozygoten Form der Erkrankung betroffen sind, machen sich frühzeitig Gefäßveränderungen bemerkbar, welche die hauptsächliche Ursache für den häufig lethalen Ausgang der Erkrankung im Kindesalter darstellen. Während die erhöhten Homocysteinspiegel bei den genetisch bedingten Formen der Hyperhomocysteinämie in der Regel auf einen Mangel an Cystathionin-β-Synthase und/oder auf eine Mutation der 5,10-Methylentetrahydrofolat-Reduktase zurückzuführen sind, können auch Veränderungen des Folsäure-, Vitamin-B6-, Vitamin-B12- und Betainstoffwechsels zu erhöhten Homocysteinspiegeln führen. Dementsprechend können auch erworbene Formen der Hyperhomocysteinämie auftreten. Beispielsweise können Niereninsuffizienzen oder ein Mangel an Folsäure, Cobalamin und/oder Pyridoxin bzw. Metaboliten davon zu erhöhten Homocysteinspiegeln führen. Insbesondere bei älteren Menschen gilt ein derartiger Vitaminmangel, der durch eine unzureichende Zufuhr oder durch Malabsorption verursacht werden kann, als die häufigste Ursache von erworbener Hyperhomocysteinämie.

Vitamin B12 ist erforderlich, um eine 1-Kohlenstoffeinheit von Folsäure auf Homocystein zu übertragen und dieses in Methionin zu überführen. Vitamin B6 ist an einem weiteren Stoffwechselweg für den Abbau von überschüssigem Homocystein beteiligt.

Es wurde bereits vorgeschlagen, verschiedenste Folsäure, Vitamin B6 und Vitamin B12 enthaltende Vitaminpräparate zur Senkung erhöhter Homocysteinspiegel einzusetzen.

Beispielsweise beschreibt die US 5,932,624 ein Mittel, das 500 µg Folsäure, 25 µg Vitamin B12 und 10 mg Vitamin B6 enthält. In Abhängigkeit vom Patientenzustand sollen im Allgemeinen 300 bis 2000 µg Folsäure, 25 bis 1000 µg Vitamin B12 und 5 bis 20 mg Vitamin B6 so verabreicht werden, dass die Homocystein-Plasmaspiegel auf Normalwerte sinken.

Die in US 6,274,170 angegebene Kombination aus Vitaminen und Aspirin zur Behandlung von atherosklerotischen kardiovaskulären Erkrankungen enthält 400 bis 1000 µg Folsäure, 3 bis 25 mg Vitamin B6 und 5 bis 500 µg Vitamin B12.

Ein Multivitamin- und Mineral-Supplement, der neben einer Reihe weiterer Vitamine und essentieller Spurenelemente 800 µg Folsäure, 25 mg Vitamin B6 und 400 µg Vitamin B12 enthält, wird in der US 6,299,896 beschrieben. Auch dieses Mittel soll die Homocysteinspiegel senken können.

Eine tägliche Aufnahme von 180 bis 800 µg Folsäure, 1,6 bis 4,6 mg Vitamin B6 und 1,5 bis 4,0 µg Vitamin B12 zusammen mit β-Glukan- oder Glukomannan-haltigen Fasern wird in der US 6,210,686 empfohlen, um die Zusammensetzung an Serumlipiden zu verbessern, Homocysteinspiegel zu senken und Lipoproteine vor Oxidation zu schützen.

Zur Vorbeugung und Behandlung erhöhter Homocystein-, Cystathionin-, Methylmalonsäure- oder 2-Methylcitronsäure-Serumspiegel sollen gemäß US 6,297,224 und den dazu verwandten US 6,207,651 und US 5,563,126 Vitaminzubereitungen eingesetzt werden, die 0,4 mg oder 1,0 mg Folsäure zusammen mit 25 mg Vitamin B6 und 2,0 mg Vitamin B12 enthalten.

Es wurde nun gefunden, daß die kombinierte Anwendung von Folsäure, Vitamin B6 und Vitamin B12 in bestimmten Mengen überraschenderweise den Homocysteinspiegel noch wirksamer senkt und damit das Risiko für vaskuläre Erkrankungen noch weiter herabgesetzt werden kann als mit den bisher bekannten Kombinationen aus Folsäure, Vitamin B6 und Vitamin B12.

Gegenstand der vorliegenden Erfindung sind daher Mittel auf Basis von Folsäure, Vitamin B6 und Vitamin B12 beziehungsweise physiologisch akzeptabler Derivate und/oder Salze davon, die dadurch gekennzeichnet sind, dass die Mengenverhältnisse von Folsäure zu Vitamin B6 und von Vitamin B12 zu Vitamin B6 in einem Bereich von etwa 1:67 bis 1:150 liegen, und das Mengenverhältniss von Folsäure zu Vitamin B12 in einem Bereich von etwa 1:0,67 bis 1:1,50 liegt.

Die erfindungsgemäßen Mittel auf Basis von Folsäure, physiologisch akzeptabler Derivate oder Salze davon (zwecks Vereinfachung auch als "Folsäuren" oder "Folsäure-Komponente" bezeichnet), Vitamin B6, physiologisch akzeptabler Derivate oder Salze davon (zwecks Vereinfachung auch als "B6-Vitamine" oder "Vitamin B6-Komponente" bezeichnet) und Vitamin B12, physiologisch akzeptabler Derivate oder Salze davon (zwecks Vereinfachung auch als "B12-Vitamine" oder "Vitamin B12-Komponente" bezeichnet) bieten wesentliche Vorteile bei der Regulation von Homocysteinspiegeln und damit bei der vorbeugenden und akuten Behandlung von vaskulären Erkrankungen.

Gegenstand der vorliegenden Erfindung ist daher auch die Verwendung der erfindungsgemäßen Kombination aus Folsäure, Vitamin B6 und Vitamin B12 beziehungsweise physiologisch akzeptablen Derivaten und/oder Salzen davon zur Regulation des Homocysteinspiegels. Die Regulation betrifft insbesondere im akuten Bereich die Senkung erhöhter Homocysteinspiegel, d.h. insbesondere die Behandlung von Hyperhomocysteinämie, sowie im prophylaktischen Bereich die Vermeidung erhöhter Homocysteinspiegel und die Beibehaltung normaler Homocysteinspiegel. Mit der Regulation von Homocysteinspiegeln verbunden ist insbesondere eine prophylaktische Behandlung von Erkrankungen, die mit erhöhten Homocysteinspiegeln in Zusammenhang stehen.

Ein weiterer Gegenstand der vorliegenden Erfindung ist daher die Verwendung der erfindungsgemäßen Kombination aus Folsäure, Vitamin B6 und Vitamin B12 beziehungsweise physiologisch akzeptablen Derivaten und/oder Salzen davon zur Behandlung von Erkrankungen, die mit einem erhöhten Homocysteinspiegel in Zusammenhang stehen. Hierzu gehören insbesondere vaskuläre Erkrankungen, wie Arterioskleorse, venöse Thrombosen und arterielle Verschlüsse, foetale Schäden, wie Neuralrohrdefekte, und neurodegenerative Erkrankungen, wie bestimmte Formen der Alzheimerschen Demenz.

In diesem Sinne sind Gegenstand der Erfindung auch Mittel zur Regulation des Homocysteinspiegels und zur Behandlung von Erkrankungen, die mit erhöhten Homocysteinspiegeln in Zusammenhang stehen. Diese Mittel basieren auf der erfindungsgemäßen Wirkstoffkombination und gegebenenfalls weiteren Wirkstoffen, wobei die Wirkstoffe bzw. Wirkstoffkomponenten gemeinsam in einer Formulierung oder getrennt in wenigstens zwei oder drei verschiedenen Formulierungen formuliert sein können.

Bevorzugt sind Mittel und Verwendungen, bei denen die Mengenverhältnisse von Folsäure zu Vitamin B6 und von Vitamin B12 zu Vitamin B6 in einem Bereich von etwa 1:82 bis 1:122 liegen, und das Mengenverhältniss von Folsäure zu Vitamin B12 in einem Bereich von etwa 1:0,82 bis 1:1,22 liegt.

Von besonderem Vorteil sind Mittel und Verwendungen, bei denen das Mengenverhältnis von Folsäure zu Vitamin B6 zu Vitamin B12 etwa 1:100:1 beträgt.

Dabei beziehen sich die angegebenen Mengenverhältnisse auf Gewichtsmengen der Wirkstoffe Folsäure, Vitamin B6 und Vitamin B12, so daß für Salze und Derivate erforderlichenfalls eine entsprechende Umrechnung zu erfolgen hat. Dies gilt analog für die in der vorliegenden Beschreibung angegebene Wirkstoffanteile.

"Folsäure" bezeichnet erfindungsgemäß N-Pteroylglutaminsäure der Formel I 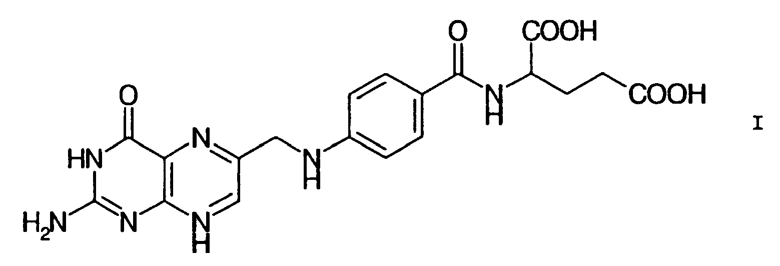 die unter diese Formel fallenden optischen Isomere sowohl als Gemische, z.B. als Racemat, als auch in Reinform, z.B. R- oder S-Enantiomere, eingeschlossen. Bevorzugt ist N-Pteroyl-L-glutaminsäure der Formel Ia

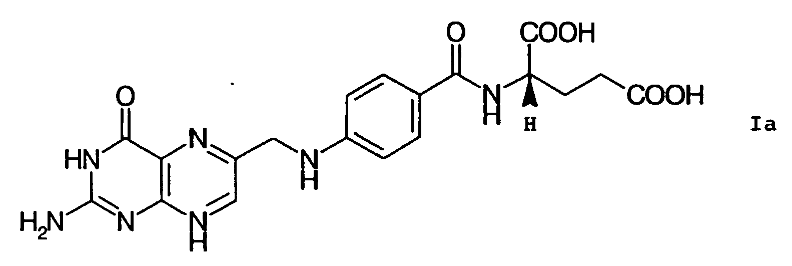

Zu den Folsäure-Derivaten gehören vor allem Folsäure-Metabolite sowie Amide und Ester der Folsäure wie auch der Metabolite. Vorteilhaft sind Amide und Ester, die unter physiologischen Bedingungen hydrolisierbar sind, wie Amide mit C₁-C₁₀-Alkylaminen oder Ester mit C₁-C₁₀-Alkoholen. Eine besondere Form der Amide sind N-Pteroylpolyglutaminsäuren.

Zu den Folsäure-Metaboliten gehören vor allem H₄-Folsäuren der Formel Ib. 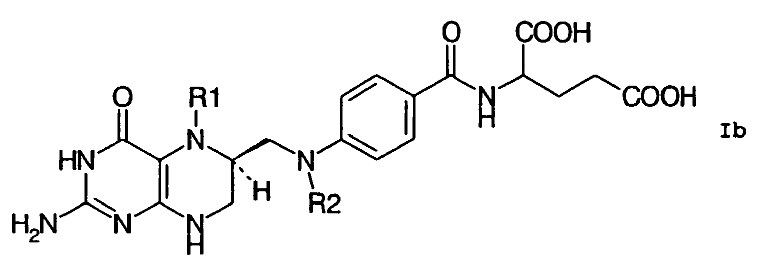 worin R1 für Wasserstoff, Methyl, -HC=O (Formyl) oder -HC=NH (Formimino) steht und R2 für Wasserstoff oder -HC=O (Formyl) steht, oder R1 und R2 zusammen genommen eine Methylen oder Methenylbrücke bilden. Die unter diese Formel fallenden optischen Isomere sind obigen Ausführungen entsprechend eingeschlossen, wobei auch hier die L-Glutaminsäure-Derivate bevorzugt sind. Insbesondere sind Tetrahydrofolsäure, 5-Methyltetrahydrofolsäure, 5,10-Methylentetrahydrofolsäure, 5-Formyltetrahydrofolsäure, 10-Formyltetrahydrofolsäure, 5-Formiminotetrahydrofolsäure und 5,10-Methenyltetrahydrofolsäure zu nennen.

Zu physiologisch akzeptablen Salzen von Folsäure bzw. Folsäure-Derivaten gehören Säure- und Basenadditionssalze sowie entsprechende Mischformen.

Zu den Säureadditionssalzen zählen Salze von Folsäure bzw. Folsäure-Derivaten mit anorganischen Säuren, wie Salzsäure, Schwefelsäure, Salpetersäure oder Phosphorsäure, oder organischen Säuren, insbesondere Carbonsäuren, z.B. Essigsäure, Weinsäure, Milchsäure, Citronensäure, Apfelsäure, Mandelsäure, Ascorbinsäure, Maleinsäure, Fumarsäure, Gluconsäure oder Sulfonsäuren, z. B. Methansulfonsäure, Benzolsulfonsäure und Toluolsulfonsäure, und dergleichen.

Zu den Basenadditionssalzen zählen Salze von Folsäure bzw. Folsäure-Derivaten mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z.B. Methylamin, Dimethylamin, Trimethylamin, Triethylamin, Ethylamin, Diethylamin, Ethylendiamin, Ethanolamin, Diethanolamin, 1-Amino-2-propanol, 3-Amino-1-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt sind Salze mit anorganischen Basen, z.B. Na-, K-, Mg-, Ca-, Zn-, Cr- und Fe-Folate.

"Vitamin B6" bezeichnet erfindungsgemäß 4,5-Bis(Hydroxymethyl)-2-methyl-3-pyridinol der Formel II 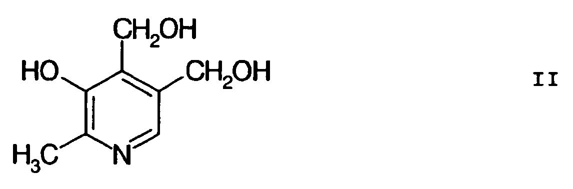 auch als Pyridoxin (INN) bezeichnet.

Zu den Vitamin B6-Derivaten gehören vor allem Pyridoxale und Pyridoxamine sowie Ester der Pyridoxine, Pyridoxale und Pyridoxamine. Vorteilhaft sind auch hier Ester, die unter physiologischen Bedingungen hydrolisierbar sind.

Insbesondere zu nennen sind in diesem Zusammenhang die Pyridoxine, Pyridoxale und Pyridoxamine der Formel IIa 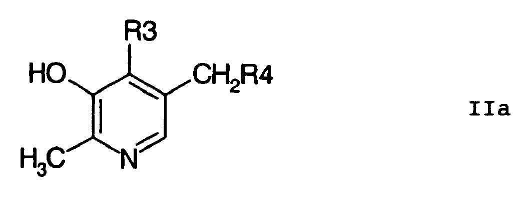 worin R³ für CH₂OH, CHO oder CH₂NH₂ steht und R⁴ für OH oder OPO₃H₂ steht.

Zu physiologisch akzeptablen Salzen von Vitamin B6 bzw. Vitamin B6-Derivaten gehören insbesondere Säureadditionssalze, z.B. mit den oben genannten anorganischen und organischen Säuren. Insbesondere ist das Hydrochlorid, vor allem Pyridoxin-HCl, zu nennen. "Vitamin B12" wird auch als Cyanocobalamin oder Cobalamin bezeichnet.

Zu den Vitamin-B12-Derivaten gehören insbesondere Cobalamine, in denen die Cyanogruppe des Cyanocobalamins durch andere Koordinationspartner des Cobalts ersetzt ist. Hierzu zählen vor allem das Hydroxocobalamin, Aquocobalamin, Nitrosocobalamin, Methylcobalamin und Adenosylcobalamin (Coenzym B12).

Zu physiologisch akzeptablen Salzen von Vitamin B12 bzw. Vitamin B12-Derivaten gehören insbesondere Säureadditionssalze z.B. mit den oben genannten anorganischen und organischen Säuren. Insbesondere ist das Acetat des Hydoxocobalamins zu nennen.

Folsäuren, B6- und B12-Vitamine sind hinlänglich bekannt und können entweder bezogen oder in an sich bekannter Weise zur Verfügung gestellt werden.

Neben den Folsäure-, Vitamin B6- und Vitamin-B12-Komponenten können die erfindungsgemäßen Mittel weitere Wirkstoffe miteinbeziehen. Bei diesen Wirkstoffen kann es sich insbesondere um solche handeln, deren Wirkung der Folsäure-, Vitamin B6- bzw. Vitamin B12-vermittelten Wirkung ähnlich ist oder diese ergänzt und die insbesondere den erfindungesgemäßen Verwendungszwecken entspricht. So kann es von Vorteil sein, zusätzlich zur erfindungsgemäßen Kombination, Homocysteinspiegel senkende Wirkstoffe, Antithrombotika, Antisklerotika und ähnliches zu verabreichen.

Assays zur Bestimmung von Homocysteinspiegeln, die normalerweise in einem Bereich von 5 bis 15 µmol/l Blutplasma liegen, sind bekannt (vgl. z.B. den eingangs geschilderten Stand der Technik). Erhöhte Homocysteinspiegel werden als Hyperhomocysteinämie bezeichnet. Mithilfe der erfindungsgemäßen Mittel können erhöhte Homocysteinspiegel gesenkt bzw. präventiv vermieden werden.

Je nach Homocysteinspiegel, werden Hyperhomocysteinämien in drei Klassen eingeteilt:

Leichte Hyperhomocysteinämien sind gekennzeichnet durch Homocysteinspiegel in einem Bereich von mehr als 15 und bis zu 30 µmol/ l Blutplasma.

Mittlere Hyperhomocysteinämien sind gekennzeichnet durch Homocysteinspiegel in einem Bereich von mehr als 30 und bis zu 100 µmol/l Blutplasma.

Hohe Hyperhomocysteinämien sind gekennzeichnet durch Homocysteinspiegel von mehr als 100 µmol/l Blutplasma.

Besondere Vorteile ergeben sich erfindungsgemäß im Hinblick auf die Behandlung von mittleren Hyperhomocysteinämien.

Die vorliegende Erfindung richtet sich insbesondere auf die Behandlung eines oder mehrerer der folgenden Krankheitsbilder:

Hereditäre Hyperhomocysteinämie. Das Krankheitsbild der hereditären Hyperhomocysteinämie ist gekennzeichnet durch genetisch bedingte Störungen des Homocystein-Stoffwechsels. Zu derartigen Stoffwechselstörungen gehören insbesondere ein Fehlen (homozygote Form) oder Mangel (heterozygote Form) der Cystathionin-β-Syrithase, ein Mangel an Methylentetrahydrofolat-Reduktase, eine mutationsbedingte Veränderung der Methylentetrahydrofolat-Reduktase zu einem thermolabilen Derivat davon sowie eine Reihe weiterer Veränderungen des Folsäure-, Vitamin-B6-, Vitamin-B12- und des Betainstoffwechsels. Zu klinischen Symptomen einer hereditären Hyperhomocysteinämie gehören Homocysteinurie, geistige Retardierung, Subluxation der Augenlinsen, Skelettanomalien und/oder vaskuläre Erkrankungen, die damit als Symptom oder Syndrom erfindungsgemäß akut oder präventiv behandelbar sind.

Erworbene Hyperhomocysteinämie. Erworbene Formen der Hyperhomocysteinämie sind in der Regel gekennzeichnet durch Mangelerscheinungen, die zu einer Akkumulation von Homocystein führen. Beispielsweise können Mängel an Folsäure und Folsäurederivaten, Vitamin B12 und Vitamin-B12-Derivaten, Vitamin B6 und Vitamin-B6-Derivaten sowie ein allgemeiner Vitaminmangel, zu erhöhten Homocysteinspiegeln führen. Dabei kann der Vitaminmangel beispielsweise durch eine unzureichende Zufuhr oder durch Malabsorption des oder der jeweiligen Vitamine bedingt sein. Auch können Medikamente, die den Folsäure-Metabolismus beeinflussen können, wie Methotrexat oder Antikonvulsiva; die den Vitamin-B12-Metabolismus beeinflussen können, wie Nitrokörper; oder die den Vitamin-B6-Metabolismus beeinflussen können, wie Theophyllin, erhöhte Homocysteinspiegel verursachen. Ferner beeinflussen Alter, Geschlecht, Zigarettenrauchen, arterielle Hypertonie, Hypercholesterinämie und Bewegungsmangel den Homocystein-Plasmaspiegel.

Weiterhin richtet sich die vorliegende Erfindung auf die Behandlung von Erkrankungen, die mit erhöhten Homocysteinspiegeln in Zusammenhang stehen, insbesondere damit einhergehen oder dadurch verursacht werden. Hierzu zählen vor allem vaskuläre Erkrankungen, foetale Mißbildungen und bestimmte neurodegenerative Erkrankungen. In diesem Indikationsbereich ist vor allem die Prävention von Bedeutung.

Unter vaskulären Erkrankungen versteht man Erkrankungen der peripheren, koronaren und zerebralen Gefäße. Insbesondere zu nennen sind Veränderungen an den Gefäßendothelzellen, eine Proliferation der Muskelzellen und/oder eine Verdickung der Gefäßintima. Erfindungsgemäß behandelbar sind damit insbesondere Arteriosklerosen, venöse Thrombosen, arterielle Verschlüsse und weitere arteriovenöse Gefäßleiden.

Foetale Mißbildungen, insbesondere Neuralrohrdefekte, können im Rahmen einer Schwangerschaft auftreten, wenn die Mutter unter erhöhten Homocysteinspiegeln leidet.

Erhöhte Homocysteinspiegel können auch an neurodegenerativen Erkrankungen, insbesondere vaskulär bedingten Formen der Altersdemenz, beteiligt sein.

Somit richtet sich die Erfindung einem besonderen Aspekt zufolge auf Verminderung des Risikos für das Auftreten der vorstehend beschriebenen vaskulären Erkrankungen, foetaler Mißbildungen und der neurodegenerativen Erkrankungen.

Die erfindungsgemäßen Mittel und Anwendungen gewinnen bei Erwachsenen mit zunehmendem Lebensalter an Bedeutung. In der Gruppe der über 40-jährigen und vor allem der über 50-jährigen bringt die Behandlung besondere Vorteile mit sich. Die erfindungsgemäße Behandlung ist insbesondere dann angezeigt, wenn Hinweise auf Arteriosklerosen, arterielle Verschlüsse, venöse Thrombosen und/oder vaskulär bedingte Formen der Altersdemenz vorliegen oder ein Risiko für diese Erkrankungen besteht. Eine weitere Gruppe, bei denen die erfindungsgemäße Behandlung besondere Vorteile mit sich bringen kann, sind Kinder mit hereditärer Hyperhomocysteinämie sowie schwangere Frauen, selbst wenn Hinweise auf vaskuläre Erkrankungen nicht vorliegen und die Homocysteinspiegel nur leicht erhöht sind.

Erfindungsgemäß wird dem zu behandelnden Individuum, vorzugsweise einem Säuger, insbesondere einem Menschen und auch einem Nutz- oder Haustier, eine wirksame Menge der erfindungesgemäßen Wirkstoffkombination aus Folsäure-Komponente, Vitamin-B6-Komponente und Vitamin-B12-Komponente, in der Regel der pharmazeutischen, tierarzneilichen oder lebensmitteltechnologischen Praxis entsprechend formuliert, verabreicht. Wirksam ist eine Menge erfindungsgemäß insbesondere dann, wenn sie eine signifkante Senkung des Homocysteinspiegels, vorteilhafterweise bis in den Normalbereich, bewirkt.

Die Behandlung erfolgt in der Regel durch einmaliges oder mehrmaliges tägliches Verabreichen einer geeigneten Dosis gegebenenfalls zusammen oder im Wechsel mit anderen Wirkstoffen oder wirkstoffhaltigen Präparaten, so dass einem zu behandelnden Individuum mit dem Gewicht eines durchschnittlichen Erwachsenen von etwa 75 kg in der Regel eine Tagesmindestdosis von etwa 0,8 mg, vorzugsweise etwa 0,9 mg und vorteilhafterweise etwa 1 mg Folsäure, von etwa 80 mg, vorzugsweise etwa 90 mg und vorteilhafterweise etwa 100 mg Vitamin B6, sowie von etwa 0,8 mg, vorzugsweise etwa 0,9 mg und vorteilhafterweise etwa 1 mg Vitamin B12 verabreicht wird. Einem anderen Aspekt zufolge beträgt die Tageshöchstdosis in der Regel etwa 1,2 mg, vorzugsweise etwa 1,1 mg und vorteilhafterweise etwa 1 mg Folsäure, etwa 120 mg, vorzugsweise etwa 110 mg und vorteilhafterweise etwa 100 mg Vitamin B6, sowie etwa 1,2 mg, vorzugsweise etwa 1,1 mg und vorteilhafterweise etwa 1 mg Vitamin B12. Bei Abweichungen vom Durchschnittsgewicht sollte die Tagesdosis entsprechend angepaßt werden. Diese Anpassung erfolgt in üblicher Weise durch den Fachmann erforderlichenfalls unter Berücksichtigung analytischer Kontrolluntersuchungen. Weiterhin können sich Abweichungen in der Tagesdosis durch den verordnenden Arzt auch aufgrund des Gesundheitszustandes des zu behandlenden Individuums ergeben.

Die Behandlung erfolgt in der Regel über einen angemessenen Zeitraum im Bereich von Tagen oder Wochen. Zweckmäßig ist eine Normalisierung der Homocysteinspiegel innerhalb eines Behandlungszeitraums von etwa 1 bis 4 Wochen. Erforderlichenfalls wird die Behandlung auch nach Normalisierung der Homocysteinspiegel fortgesetzt. Dies gilt insbesondere für heriditäre Formen der Hyperhomocysteinämie und erworbene Formen, bei denen eine ursächliche Behandlung nicht möglich ist oder keinen Erfolg gezeigt hat und das Absetzen der erfindungsgemäßen Behandlung ein erneutes Ansteigen der Homocysteinspiegel zur Folge hätte.

Die Erfindung betrifft auch die Herstellung von Mitteln zur Behandlung eines Individuums, vorzugsweise eines Säugers, insbesondere eines Menschen und auch eines Nutz- oder Haustieres.

Zu den Mitteln gehören insbesondere pharmazeutische Mittel, Nahrungsergänzungsmittel und Lebensmittel, z.B. funktionale oder diätetische Lebensmittel. Die erfindungsgemäßen Lebensmittel besitzen neben einer vorwiegend nährwertbezogenen Funktion zusätzlich eine wirkstoffbezogene Funktion, welche insbesondere die erfindungsgemäße Wirkstoffkombination betrifft. Sie werden daher als funktionale oder diätetische Lebens- oder Nahrungsmittel bezeichnet. Nahrungsergänzungsmittel dienen zur Ergänzung der täglichen Ernährung mit der erfindungsgemäßen Wirkstoffkombination, wobei die nährwertbezogene Funktion des Nahrungsergänzungsmittels für sich genommen in den Hintergrund tritt.

Einem Aspekt zufolge betrifft die vorliegende Erfindung Formulierungen, enthaltend
i) wenigstens einen Wirkstoff aus der Folsäure-Gruppe (Folsäure, physiologisch akzeptable Derivate und/oder Salze davon),
ii) wenigstens einen Wirkstoff aus der Vitamin-B6-Gruppe (Vitamin B6, physiologisch akzeptable Derivate und/oder Salze davon), und
iii) wenigstens einen Wirkstoff aus der Vitamin-B12-Gruppe (Vitamin B12, physiologisch akzeptable Derivate und/oder Salze davon), sowie
gegebenenfalls wenigstens einen weiteren Wirkstoff und eine Formulierungsgrundlage, in den erfindungsgemäß angegebenen Mengenverhältnissen.

Somit umfaßt die Wirkstoffkombination im Sinne der Erfindung als Wirkstoffkomponente i) Folsäure, ein physiologisch akzeptables Derivat und/oder Salz davon. Gemische dieser Formen sind möglich, jedoch nur in bestimmten Fällen in Betracht zu ziehen. Gemäß einer besonderen Ausführungsform besteht die Wirkstoffkomponente i) aus wenigstens 90 Gew.-% Folsäure.

Weiterhin umfaßt die Wirkstoffkombination im Sinne der Erfindung als Wirkstoffkomponente ii) Vitamin B6, ein physiologisch akzeptables Derivat und/oder Salz davon. Gemische dieser Formen sind ebenfalls möglich, jedoch nur in bestimmten Fällen in Betracht zu ziehen. Gemäß einer besonderen Ausführungsform besteht die Wirkstoffkomponente ii) aus wenigstens 90 Gew.-% Pyridoxin-HCI.

Weiterhin umfaßt die Wirkstoffkombination im Sinne der Erfindung als Wirkstoffkomponente iii) Vitamin B12, ein physiologisch akzeptables Derivat und/oder Salz davon. Gemische dieser Formen sind ebenfalls möglich, jedoch nur in bestimmten Fällen in Betracht zu ziehen. Gemäß einer besonderen Ausführungsform besteht die Wirkstoffkomponente iii) aus wenigstens 90 Gew.-% Cobalamin.

Der Anteil der Wirkstoffkombination an der Formulierung ist größer als ein gegebenenfalls in natürlichen Quellen, insbesondere Lebensmitteln, vorhandener Anteil. In diesem Sinne sind die erfindungsgemäßen Mittel im Hinblick auf die Wirkstoffkombination angereichert. Der Anteil der Wirkstoffkombination aus i), ii) und iii) an der Formulierung beträgt vorzugsweise mindestens etwa 0,01 Gew.-%, vorteilhafterweise mindestens etwa 0,05 Gew.-% und insbesondere mindestens etwa 0,1 Gew.-%. Im Falle eines pharmazeutischen Mittels liegt der Anteil in der Regel bei etwa 1 bis 60 Gew.-%, vorzugsweise bei etwa 5 bis 35 Gew.-% und insbesondere bei etwa 10 bis 30 Gew.-%, im Falle eines Nahrungsergänzungsmittels und vor allem bei Lebensmitteln gegebenenfalls entsprechend niedriger, wenn die Formulierung in größeren Mengen zugeführt wird. Vorzugsweise enthalten die Formulierungen die angegebene Tagesdosis.

Angaben in Gew.-% beziehen sich, sofern nicht anderes angegeben ist, auf das Gesamtgewicht der Formulierung.

Die Formulierungsgrundlage erfindungsgemäßer pharmazeutischer Formulierungen enthält physiologisch akzeptable Hilfsstoffe. Physiologisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z.B. DAB, Ph. Eur., BP, NF) gelisteten, und auch andere Hilfstoffe, deren Eigenschaften einer physiologischen Anwendung nicht entgegenstehen. Hilfsstoffe im erfindungsgemäßen Sinne können auch einen Nährwert besitzen und deshalb allgemein als Nahrungskomponente verwendet werden. Auch Nährstoffe, insbesondere essentielle Nährstoffe, können dazu gehören.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Nahrungskomponenten enthalten in der Regel eine oder mehrere Aminosäuren, Kohlenhydrate oder Fette und sind für die menschliche und/oder tierische Ernährung geeignet. Sie umfassen Einzelkomponenten, häufig pflanzliche aber auch tierische Produkte, insbesondere Zucker gegebenenfalls in Form von Sirups, Fruchtzubereitungen, wie Fruchtsäfte, Nektar, Fruchtpulpen, Pürees oder getrocknete Früchte, beispielsweise Apfelsaft, Grapefruitsaft, Orangensaft, Apfelmus, Tomatensauce, Tomatensaft, Tomatenpüree; Getreideprodukte, wie Weizenmehl, Roggenmehl, Hafermehl, Maismehl, Gerstenmehl, Dinkelmehl, Maissirup, sowie Stärken der genannten Getreide; Milchprodukte, wie Milcheiweiß, Molke, Joghurt, Lecithin und Milchzucker.

Zu den essentiellen Nährstoffen zählen insbesondere Vitamine, Provitamine, Spurenelemente, Aminosäuren und Fettsäuren. Als essentielle Aminosäuren seien genannt Isoleucin, Leucin, Lysin, Methionin, Phenylalanin, Threonin, Tryptophan und Valin. Dazu gehören auch semi-essentielle Aminosäuren, die beispielsweise in Wachstumsphasen oder Mangelzuständen zugeführt werden müssen, wie Arginin, Histidin, Cystein und Tyrosin. Als Spurenelemente seien genannt: essentielle Spurenelemente, deren Notwendigkeit für den Menschen erwiesen ist und deren Mangel zur Manifestation klinischer Symptome führt: Eisen, Kupfer, Zink, Chrom, Selen, Calcium, Magnesium, Kalium, Lithium, Cobalt, Molybdän, Iod, Silicium, Fluor, Mangan. Ebenso Elemente, deren Funktion für den Menschen noch nicht genügend gesichert ist: Zinn, Nickel, Vanadium, Arsen, Mangan. Als für den Menschen essentielle Fettsäuren seien genannt: Linolsäure und Linolensäure. Eine umfassende Aufzählung von Vitaminen findet sich in "Referenzwerte für die Nährstoffzufuhr", 1. Auflage, Umschau Braus Verlag, Frankfurt am Main, 2000, herausgegeben von der Deutschen Gesellschaft für Ernährung.

Die Summe aus Wirkstoffkomponente und Formulierungsgrundlage beträgt in der Regel 100 Gew.-%.

Beispiele geeigneter Formulierungen zur Nahrungsergänzung sind Kapseln, Tabletten, Pillen, Pulverbeutel, Flüssigampullen und Fläschchen mit Tropfeinsätzen, im übrigen die nachfolgend genannten Arzneiformen.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Wirkstoffe verwendet werden. Ferner können auch Liposomen oder Mikrosphären zur Anwendung kommen.

Lebensmitteltechnische Formulierungen haben in der Regel die übliche Form und werden bevorzugt in Form von Kleinkindnahrung, Frühstückszubereitungen, vor allem in Form von Müslis oder Riegeln, Sportlerdrinks, Komplettmahlzeiten, insbesondere im Rahmen von total bilanzierten Diäten, diätetische Zubereitungen, wie Diätdrinks, Diätmahlzeiten und Diätriegel, angeboten.

Die Formulierungen werden vorzugsweise auf oralem, sie können aber auch insbesondere im Breich der Arzneimittel auf rektalem, transdermalem, subkutanem, intravenösem, intramuskulärem oder intranasalem Weg verabreicht werden.

Bei der Herstellung der Zusammensetzungen werden die Wirkstoffe gewöhnlich mit einem geeigneten Hilfsstoff, in diesem Fall auch als Exzipient zu bezeichnen, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z.B. eine Granulierung, Komprimierung und ähnliches.

Insbesondere können die Wirkstoffkomponenten gemeinsam formuliert werden. Sie können aber auch zunächst getrennt verarbeitet und anschließend in einer kompartimentierten, z.B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Die vorliegende Erfindung wird anhand der nachfolgenden Beispiele näher erläutert, ohne darauf beschränkt zu sein.

### Beispiel 1

### Pharmazeutische Mittel

### a) Weichgelatine-Kapsel

**(Folsäure 1 mg + Vitamin B6 100 mg + Vitamin B12 1 mg)**

| Füllung: | |
|---|---|
| Folsäure | 1 mg |
| Vitamin B6 | 100 mg |
| Vitamin B12 | 1 mg |
| Sojaöl (raff.) | 440 mg |
| Sojalecithin (E322) | 50 mg |
| Hochdisperses Siliciumdioxid | 5 mg |

| Kapselhülle: | |
|---|---|
| Gelatine | 303 mg |
| Glycerol 85 % | 87 mg |
| Sorbitol 70 % | 77 mg |
| gereinigtes Wasser | 52 mg |
| Eisenoxidpigment Braun 75 ( E 172) | 3 mg |

### b) Tablette

**(Folsäure 1 mg + Vitamin B6 100 mg + Vitamin B12 1 mg)**

| | |
|---|---|
| Folsäure | 1 mg |
| Vitamin B6 | 100 mg |
| Vitamin B12 | 1 mg |
| Milchzucker | 127,5 mg |
| Magnesiumstearat | 5 mg |
| Talcum | 23,75 mg |
| Mikrokristalline Cellulose | 81 mg |

### Beispiel 2

### Klinische Untersuchung

Das Risiko, an einem kardiovaskulären Leiden zu erkranken, nimmt mit einer Erhöhung des Homocysteinspiegels um 5 µmol/l bei Männern um das etwa 1,6-fache, bei Frauen um das etwa 1,8-fache zu. Diese Zunahme des Erkrankungsrisikos ist vergleichbar mit einer durch eine Erhöhung des Cholesterinspiegels um 0,5 mmol/l hervorgerufenen Zunahme des Erkrankungsrisikos.

Verabreicht man den betroffenen Patienten hingegen eine Kombination aus täglich 1 mg Folsäure, 100 mg Vitamin B6 und 1 mg Vitamin B12, so sinkt das Erkrankungsrisiko für Arteriosklerose mindestens um den Faktor 2 bis 4.

## Patentansprüche

1. Mittel auf Basis von Folsäure, Vitamin B6 und Vitamin B12 beziehungsweise physiologisch akzeptabler Derivate und/oder Salze davon, **dadurch gekennzeichnet, dass** die Gewichtsmengenverhältnisse von Folsäure : Vitamin B6 und von Vitamin B12 : Vitamin B6 in einem Bereich von etwa 1 : 67-150, sowie von Folsäure : Vitamin B12 in einem Bereich von 1 : 0,67-1,50 liegen.

2. Mittel nach Anspruch 1, **dadurch gekennzeichnet, dass** die Gewichtsmengenverhältnisse von Folsäure : Vitamin B6 und von Vitamin B12 : Vitamin B6 in einem Bereich von etwa 1 : 82-122, sowie von Folsäure : Vitamin B12 in einem Bereich von 1 : 0,82-1,22 liegen.

3. Mittel nach Anspruch 1, **dadurch gekennzeichnet, dass** das Gewichtsmengenverhältnis von Folsäure : Vitamin B6 : Vitamin B12 etwa 1 : 100 : 1 beträgt.

4. Verwendung von Folsäure, Vitamin B6 und Vitamin B12 beziehungsweise physiologisch akzeptabler Derivate und/oder Salze davon zur Herstellung eines Mittels zur Regulation von Homocysteinspiegeln, **dadurch gekennzeichnet, dass** die Gewichtsmengenverhältnisse von Folsäure : Vitamin B6 und von Vitamin B12 : Vitamin B6 in einem Bereich von etwa 1 : 67-150, sowie von Folsäure : Vitamin B12 in einem Bereich von 1 : 0,67-1,50 liegen.

5. Verwendung von Folsäure, Vitamin B6 und Vitamin B12 beziehungsweise physiologisch akzeptabler Derivate und/oder Salze davon zur Herstellung eines Mittels zur Behandlung vaskulärer Erkrankungen, **dadurch gekennzeichnet, dass** die Gewichtsmengenverhältnisse von Folsäure : Vitamin B6 und von Vitamin B12 : Vitamin B6 in einem Bereich von etwa 1 : 67-150, sowie von Folsäure : Vitamin B12 in einem Bereich von 1 : 0,67-1,50 liegen.

6. Verwendung nach Anspruch 5, wobei die vaskuläre Erkrankung eine Arteriosklerose, ein arterieller Verschluss, eine venöse Thrombose oder eine vaskulär bedingte Form der Altersdemenz ist.

7. Verwendung von Folsäure, Vitamin B6 und Vitamin B12 beziehungsweise physiologisch akzeptabler Derivate und/oder Salze davon zur Herstellung eines Mittels zur Behandlung von schwangeren Frauen, **dadurch gekennzeichnet, dass** die Gewichtsmengenverhältnisse von Folsäure : Vitamin B6 und von Vitamin B12 : Vitamin B6 in einem Bereich von etwa 1 : 67-150, sowie von Folsäure : Vitamin B12 in einem Bereich von 1 : 0,67-1,50 liegen.

8. Verwendung nach einem der Ansprüche 5 bis 7, wobei die Tagesdosis 0,8 mg bis 1,2 mg Folsäure, 80 mg bis 120 mg Vitamin B6 sowie 0,8 mg bis 1,2 mg B12 beträgt.

9. Verwendung nach einem der Ansprüche 5 bis 7, wobei die Tagesdosis 0,9 mg bis 1,1 mg Folsäure, 90 mg bis 110 mg Vitamin B6 sowie 0,9 mg bis 1,1 mg Vitamin B12 beträgt.

10. Verwendung nach einem der Ansprüche 5 bis 7, wobei die Tagesdosis 1 mg Folsäure, 100 mg Vitamin B6 sowie 1 mg Vitamin B12 beträgt.

## Claims

1. A composition based on folic acid, vitamin B6 and vitamin B12 or physiologically acceptable derivatives and/or salts thereof, **characterized in that** the quantitative ratios of folic acid to vitamin B6 and of vitamin B12 to vitamin B6 are in a range of about 1 : 67-150 by weight, and of folic acid to vitamin B12 are in a range of 1 : 0.67-1.50 by weight.

2. The composition as claimed in claim 1, **characterized in that** the quantitative ratios of folic acid to vitamin B6 and of vitamin B12 to vitamin B6 are in a range of about 1 : 82-122 by weight, and of folic acid to vitamin B12 are in a range of 1 : 0.82-1.22 by weight.

3. The composition as claimed in claim 1, **characterized in that** the quantitative ratio of folic acid to vitamin B6 to vitamin B12 is about 1 : 100 : 1 by weight.

4. The use of folic acid, vitamin B6 and vitamin B12 or physiologically acceptable derivatives and/or salts thereof for producing a composition for regulating homocysteine levels, **characterized in that** the quantitative ratios of folic acid to vitamin B6 and of vitamin B12 to vitamin B6 are in a range of about 1 : 67-150 by weight, and of folic acid to vitamin B12 are in a range of 1 : 0.67-1.50 by weight.

5. The use of folic acid, vitamin B6 and vitamin B12 or physiologically acceptable derivatives and/or salts thereof for producing a composition for treating vascular disorders, **characterized in that** the quantitative ratios of folic acid to vitamin B6 and of vitamin B12 to vitamin B6 are in a range of about 1 : 67-150 by weight, and of folic acid to vitamin B12 are in a range of 1 : 0.67-1.50 by weight.

6. The use as claimed in claim 5, where the vascular disorder is an arteriosclerosis, an arterial occlusion, a venous thrombosis or a vascular form of dementia in the elderly.

7. The use of folic acid, vitamin B6 and vitamin B12 or physiologically acceptable derivatives and/or salts thereof for producing a composition for treating pregnant women, **characterized in that** the quantitative ratios of folic acid to vitamin B6 and of vitamin B12 to vitamin B6 are in a range of about 1 : 67-150 by weight, and of folic acid to vitamin B12 are in a range of 1 : 0.67-1.50 by weight.

8. The use as claimed in any of claims 5 to 7, where the daily dose is from 0.8 mg to 1.2 mg of folic acid, from 80 mg to 120 mg of vitamin B6 and from 0.8 mg to 1.2 mg of B12.

9. The use as claimed in any of claims 5 to 7, where the daily dose is from 0.9 mg to 1.1 mg of folic acid, from 90 mg to 110 mg of vitamin B6 and from 0.9 mg to 1.1 mg of vitamin B12.

10. The use as claimed in any of claims 5 to 7, where the daily dose is 1 mg of folic acid, 100 mg of vitamin B6 and 1 mg of vitamin B12.

## Revendications

1. Produit à base d'acide folique, de vitamine B6 et de vitamine B12, respectivement de dérivés et/ou de sels physiologiquement acceptables de ces substances, **caractérisé en ce que** les rapports quantitatifs en poids de l'acide folique sur la vitamine B6 et de la vitamine B12 sur la vitamine B6 se situent dans une plage de 1 : 67 à 150 environ et celui de l'acide folique sur la vitamine B12 dans une plage de 1 : 0,67 à 1,50.

2. Produit selon la revendication 1, **caractérisé en ce que** les rapports quantitatifs en poids de l'acide folique sur la vitamine B6 et de la vitamine B12 sur la vitamine B6 se situent dans une plage de 1 : 82 à 122 environ et celui de l'acide folique sur la vitamine B12 dans une plage de 1 : 0,82 à 1,22.

3. Produit selon la revendication 1, **caractérisé en ce que** le rapport quantitatif en poids acide folique : vitamine B6 : vitamine B12 est d'environ 1 : 100 : 1.

4. Utilisation d'acide folique, de vitamine B6 et de vitamine 812, respectivement de dérivés et/ou de sels physiologiquement acceptables de ces substances, pour préparer un produit destiné à réguler les taux d'homocystéine, **caractérisée en ce que** les rapports quantitatifs en poids de l'acide folique sur la vitamine B6 et de la vitamine B12 sur la vitamine B6 se situent dans une plage de 1 : 67 à 150 environ et celui de l'acide folique sur la vitamine B12 dans une plage de 1 : 0,67 à 1,50.

5. Utilisation d'acide folique, de vitamine B6 et de vitamine 812, respectivement de dérivés et/ou de sels physiologiquement acceptables de ces substances, pour préparer un produit destiné au traitement de pathologies vasculaires, **caractérisée en ce que** les rapports quantitatifs en poids de l'acide folique sur la vitamine B6 et de la vitamine B12 sur la vitamine B6 se situent dans une plage de 1 : 67 à 150 environ et celui de l'acide folique sur la vitamine B12 dans une plage de 1 : 0,67 à 1,50.

6. Utilisation selon la revendication 5, dans laquelle la pathologie vasculaire est une artériosclérose, une occlusion artérielle, une thrombose veineuse ou une forme d'origine vasculaire de la démence sénile.

7. Utilisation d'acide folique, de vitamine B6 et de vitamine B12, respectivement de dérivés et/ou de sels physiologiquement acceptables de ces substances, pour préparer un produit destiné au traitement de femmes enceintes, **caractérisée en ce que** les rapports quantitatifs en poids de l'acide folique sur la vitamine B6 et de la vitamine B12 sur la vitamine B6 se situent dans une plage de 1 : 67 à 150 environ et celui de l'acide folique sur la vitamine B12 dans une plage de 1 : 0,67 à 1,50.

8. Utilisation selon l'une des revendications 5 à 7, dans laquelle la dose journalière est de 0,8 mg à 1,2 mg d'acide folique, 80 mg à 120 mg de vitamine B6 et 0,8 mg à 1,2 mg de vitamine B12.

9. Utilisation selon l'une des revendications 5 à 7, dans laquelle la dose journalière est de 0,9 mg à 1,1 mg d'acide folique, 90 mg à 110 mg de vitamine B6 et 0,9 mg à 1,1 mg de vitamine B12.

10. Utilisation selon l'une des revendications 5 à 7, dans laquelle la dose journalière est de 1 mg d'acide folique, 100 mg de vitamine B6 et 1 mg de vitamine B12.
